# EUROPEAN PATENT APPLICATION

(11) **EP 2 052 630 A1**
(43) Date of publication of application: **29.04.2009**
(21) Application number: 08170092.4
(22) Date of filing: 01.08.2003
(51) Int. Cl.: A23L 1/305, A23L 1/304, A23L 1/30, A23L 1/32

(54) **Calcium Absorption Enhancer**

(30) Priority: 06.08.2002 EP 02017497
(62) Divisional of application: 03784137.6
(71) Applicant: Nestec S.A., 1800 Vevey (CH)
(72) Inventor: Kastenmayer, Peter, 1800 Vevey (CH); Barclay, Denis, 1321 Arnex-sur-Orbe (CH); Offord Cavin, Elizabeth, 1820 Montreux (CH); Pridmore-Merten, Sylvie, 1025 ST. SULPICE (CH)
(74) Representative: Marquardt, Ulf

(57) **Abstract**

The invention relates to a calcium absorption enhancer comprising as an active ingredient calcium and at least one of the components selected from the group of egg white, egg white proteins and isoflavones, as well as to an orally ingestible composition comprising it, and its use for attenuating obesity.

## Description

### Field of the invention

The invention relates to the combined use of egg white and calcium in food products, as well as to food products comprising the combination of egg white and calcium.

### Background of the invention

The importance of dietary calcium (Ca) intake is becoming increasingly recognized, not only for skeletal health, but also for the prevention of hypertension and colon cancer. In some industrialized countries, Ca intakes are not only often inadequate but also declining, mainly because consumption of milk and other dairy products is decreasing. At the same time prevalence of' osteoporosis, a disease characterised by reduced bone mass (BMD) and micro-architectural deterioration of bone tissue, with consequent increased fracture risk, is rising. (Barclay, 2001).
During skeletal growth and maturation, i.e. until the age of the early twenties in humans Ca accumulates in the skeleton at an average of 150 mg/day until peak bone mass is reached. Dulling maturity the body - and therefore the skeleton - is more or less in Ca equilibrium. From the age of 50 in men and from menopause in women, bone balance becomes negative and bone is lost from all skeletal sites. Due to more rapid bone loss during early menopause and lower peak bone mass, women have an increased fracture risk compared to men.

Consequently, it is of high interest for the public health to find solutions for people to reach a high peak bone mass. It is also of high interest for public health that people who have already reached their peak bone mass maintain it throughout life and minimise the risk of bone loss with age, particularly in women following the menopause.

Adequate Ca intake from the diet is critical to achieve optimal peak bone mass, and to reduce the rate of bone loss associated with aging (Flynn & Cashman, 1999). Milk and milk products are the most important dietary sources of Ca for most people in western countries, with cereal products, fruits and vegetables each making a much smaller contribution. However only a fraction of the Ca ingested with food is absorbed and utilised by the body for metabolic functions. This part is defined as the bioavailable part of Ca in foods.

Ca in food occurs as salts or associated with other dietary constituents in the form of complexes of Ca ions. Ca must be released in soluble, and probably ionised, form before it can be absorbed. Ca is absorbed in the intestine by two routes - transcellular and paracellular. The transcellular route involves active transport of Ca by the mucosal transport protein, calbindin and is saturable and subject to physiological and nutritional regulation via vitamin D. The paracellular route involves passive Ca transport through gap junctions between mucosal cells; it is non-saturable and essentially independent of nutritional and physiological regulation, and is concentration-dependent. Most of Ca absorption in humans occurs in the small intestine, but there is some evidence for a small colonic component.
On average, between 10 and 30% of the Ca is absorbed from a mixed diet by healthy adults. The efficiency of intestinal Ca absorption is influenced by a variety of physiological factors. Reduced efficiency in healthy individuals is observed with increasing age, menopause and vitamin D deficiency. Efficiency of absorption is increased with vitamin D excess, Ca and P deficiency and pregnancy and lactation.
Ca absorption is also influenced by a number of dietary factors. These include habitual Ca intake, Ca content of the meal, ingestion of Ca with food or without food, chemical form of Ca and gastrointestinal interaction with enhancers or inhibitors present in food.
There has been considerable research effort to identify inhibitors and enhancers of Ca absorption in food with the aim to improve Ca absorption.

Two well-recognised inhibitors of Ca absorption in food are oxalate and phytate. Thus, vegetables that are rich in oxalate have a low Ca bioavailability (e.g. spinach), whereas Ca absorption is higher from low-oxalate vegetable (e.g. kale and watercress). The inhibitory effect of Ca may be explained by the extremely low solubility of Ca oxalate, which makes the Ca unavailable in the intestine. Similarly it was shown that Ca absorption from high-phytate soybeans was lower than from low-phytate soybeans. The inhibitory effect of phytate may be explained by its capacity to form strong complexes with Ca in the small intestine.

A number of food constituents have been suggested as potential enhancers of Ca absorption, such as non-digestible oligosaccharides and individual milk components, e.g. lactose and casein phosphopeptides.
Studies in animals showed that enhancement of Ca absorption can be achieved by addition of non-digestible oligosaccharides to the diet. These oligosaccharides are largely resistant to human digestive enzymes, and upon reaching the colon intact, they are fermented by the colonic microflora. The short chain fatty acids thus produced reduce colonic pH, resolubilising the insoluble Ca complexes and making Ca available by passive diffusion in the colon. Several studies confirmed the enhancing effect in humans, indicating however that at moderate doses a more pronounced effect is seen only subjects which have high Ca requirements such as adolescent and postmenopausal women. Oligosaccharides for which increased Ca absorption has been shown in humans include fructooligosaccharides (FOS), galactooligosaccharides (GOS) and lactulose.

Ca is generally well absorbed from milk and diary products in humans. This has in part been explained by the positive effect of lactose and casein phosphopeptides on Ca absorption.
Animal studies provide strong evidence that lactose increases Ca absorption and retention. In human infants, Ca absorption is significantly higher from soy based infant formula containing lactose than from placebo. In adults, however, lactose only has an effect on Ca absorption in β-galactosidase-deficient subjects, so that it is now concluded that there is no effect in the healthy adult population.
Caseinophosphopetides (CPP) are produced in vivo and industrially by the action of proteinases on milk casein. They have been shown to possess Ca binding capacity and to maintain Ca in solution at neutral and alkaline pH. Animal studies indicated that CCP might enhance Ca bioavailability during skeletal development and prevent bone loss in older animals. Studies in humans gave somewhat inconsistent results. Whereas CPP improved Ca absorption from a standard meal no effect was found from bread meals containing low and high phytate. Similarly the addition of CCP increased Ca absorption in adults from lice-based gruel, whereas no effect was seen from a whole grain cereal. These results might indicate that the matrix of the food plays an important role and effect of CCP is restricted to foods with a moderate to low content inhibitors such as phytate.

Likewise, even fat might theoretically be viewed as an enhancer since it is known to slow gastric emptying. However, using multiple regression methods, no effect of even large variations in fat intake on absorption fraction in an observational study of middle-aged women has been found.

To resume, calcium is usually well absorbed from milk. This can be in part attributed to calcium binding proteins such as phosphoproteins, which may keep the calcium soluble for absorption in the intestine.

Processing of proteins via heat treatment with sugars, the so-called Maillard reaction is another approach to obtain proteins, which have good Ca binding properties.
Aoki et al., 1994 (Bioscience, Biotechnology, and Biochemistry, 58 (9) 1727-1728, 1994) in an article titled "Whey protein and egg white protein-glucose 6-phosphate conjugates with calcium phosphate-solubilizing properties", made an attempt to solve the above-mentioned problem, by reacting whey and egg white protein with glucose-6-phosphate (G6P) to produce calcium phosphate-solubilizing proteins, which could enhance Ca absorption in the intestine. The brown colour of whey protein-G6P conjugate developed faster than that of the egg white protein-G6P conjugate. No precipitate of calcium phosphate was formed in the presence of 2% protein-G6P conjugate in the solution containing 30mM calcium, 22mM phosphate, and 10mM citrate at pH 6.7. The effect of these product on Ca absorption in vivo in animals or humans has however not be demonstrated.

Nevertheless, and despite the work achieved by many scientific teams worldwide, no satisfactory solution have been proposed to resolve the problem of calcium deficiency, and only few ingredients have been established to improve Ca bioavailability from foods.

Consumption of soy diets are associated with many beneficial effects including lower risk of osteoporosis, cardiovascular disease, cancer, and relief of menopausal symptoms. These effects have been largely attributed to the isoflavones alone or as part of the soy protein isolate. Several lines of evidence suggest a bone protective effect of soy and its protein and/or isoflavone components, including human, animal and cellular studies. Human epidemiological studies in cross-sections of the Japanese or Chinese populations indicate a positive correlation between the intake of soy products (estimated isoflavone intake of around 30 - 70 mg/day) and an improved bone mineral density (BMD) in postmenopausal women, especially at the lumbar spine. In two recent intervention studies in American postmenopausal women, isoflavones given at doses of 80 - 90 mg/day for 6 months prevented bone loss of the lumbar spine. Soy extracts or pure isoflavones show an osteoprotective effect in the ovariectomized rat model of menopausal bone loss.

The mechanism of the osteoprotective effect of soy and its components is not yet clearly elucidated. The major hypothesis is that the isoflavones are the responsible component and they act in an estrogen-like fashion to alleviate the loss of estrogen at the menopause either by improving calcium balance or by a direct effect on the bone cells. However, many of the animal and human studies have been done on soy products or soy protein containing various levels of isoflavones and few have been done with pure isoflavones, therefore it is difficult to attribute the effects with certitutude to the isoflavones rather than other components of soy such as the protein. Nevertheless, pure isoflavones do reverse bone loss in the rat model and, at the cellular level, they show weak estrogenic activity, inhibition of osteoclast (bone resorbing) activity and even a potential to stimulate bone formation.

A positive effect of soy diets on bone is the conservation of calcium in subjects consuming a soy protein rather than animal protein diet This is probably due to the lower sulfur-containing amino acids content of soy protein compared with animal protein. An increased excretion of sulfate originating from sulfur-containing amino acids inhibits the tubular calcium reabsorption.

### Summary of the invention

We have now found that giving calcium in parallel with egg white considerably enhances calcium absorption. Accordingly, the invention relates to the association of calcium and egg white, and food product containing so. In another aspect, the invention relates to a food product having the ability to enhance calcium absorption, and to a food product having the ability to reduce obesity.

### Detailed description of the invention

In the present application, the term "food product" is intended to encompass any consumable matter. Hence, it may be a product intended for the consumption by humans, but the term also encompasses products to be consumed by animals, for example pets, such as dogs, cats, rabbits, guinea pigs, mice, rats, birds (for example parrots), reptiles and fish (for example goldfish). However, the term also includes food to be consumed by other domesticated animals, such as livestock, for example, cattle, horses, pigs, sheep, goats, buffaloes, camels, and the like.

Within the context of this specification, the term "functional ingredient" refers more particularly to the ILSI European definition that states that a functional food can be regarded as "functional" if it is satisfactory demonstrated to affect beneficially one or more targets in the body, beyond adequate nutritional effects in a way that is either an improved state of health and well being and/or reduction of risk of disease. In particular, functional ingredients are nutritive substances that can be added to foods in controlled quantities in order to fulfil a specific physiological function to promote the health and well being of the consumer. The functional ingredients may include ingredients having active effects in dental or medical hygiene, bone health, digestive aid, intestinal protection, siress relief, throat sooters, breath fresheners, etc.

Accordingly, we have surprisingly found that by giving either egg white or soy isoflavones or a combination of isoflavones and egg white together with calcium, the absorption of calcium was significantly enhanced. Although not wishing to be bound by theory, we believe that an explanation of this phenomenon lies in two facts: the first one is that egg white and isoflavones stabilise the emulsion of calcium, preventing it from precipitation; the second one is that egg white and its proteins are transformed into peptides during digestion, and these peptides help to keep calcium soluble in the intestine. The third hypothesis concerns isoflavones:
Estrogen plays an important role in the maintainance of calcium balance, although the mechanism of action is still poorly understood. Depletion of estrogen at the menopause results in a decline in Ca absorption, increased urinary Ca losses and subsequent bone loss. Calcium absorption in postmenopausal osteoporosis is improved by hormone replacement therapy, particularly in combination with calcitriol. It is not clear if the menopausal changes in calcium metabolism are the cause or result of postmenopausal bone loss. In the latter case, estrogen deficiency would lead to increased bone resorption and an indirect effect on Ca metabolism via calciotropic hormones. However, a number of animal studies give evidence for a direct positive action of estrogen on gastrointestinal absorption and renal tubular reabsorption of calcium Estrogen receptors are present in the intestine and kidney and this could explain an estrogen regulation of vitamin D receptors and calbindin protein in these tissues.

Soy isoflavones are weak estrogens. They are 1000 fold less potent than the natural estrogen, estradiol (Markiewicz, et al. 1993). However, in women consuming a soy diet, circulating plasma levels of isoflavones are 1000 fold higher than estradiol and result in physiological effects. By analogy with estrogen, it is postulated that soy isoflavones, which bind to estrogen receptors (ERs), though with a higher affinity for ERβ than ERα, may modulate gastrointestinal absorption and renal tubular reabsorption of calcium.

Egg white, and especially hen's egg white, contains three major proteins, namely ovotransferrin, ovomuccoid and ovalbumin. In the present invention, hen's egg white is preferred, but egg whites from other species can also be used. For example, it is possible to use egg white from pigeon, ostrich, or quail, among others, because they contain the same proteins approximately in the same proportions.

Phyto-oestrogens occur naturally in many fruits, vegetables, and whole grain products. Soy and soy products are the most significant dietary source of isoflavones, which, together with coumestans and lignans, are broadly defined as phyto-oestrogens due to the similarity in their structure to the natural oestrogen, oestradiol-17β, and their weak oestrogenicity (Reinh and Block, 1996; Kurzer and Xu, 1997). The mean dietary intake of soy isoflavones in Asian populations consuming soy based diets ranges from 20 - 40 mg isoflavones/day , with upper percentile consumer intakes of 70 mg/day (approx. 1mg/kg body wt/day). Such dietary intake leads to circulating plasma levels of isoflavones in the range of 0.5 to 5 µM. The major isoflavones in soy are genistein, daidzein and glycitein. Isoflavones exist mainly as glycosides (acetyl-glycoside or malonyl-glycoside) in the soybean and in non-fermented foods. The glycosides are converted to the aglycone form through the action of β-glycosidases via the gut microflora.

The first aspect of the invention lies in giving together egg white and calcium or isoflavones and calcium, in order to enhance calcium absorption. Accordingly, egg white and isoflavones are preferably given in powder form The egg white preferably contains either all the proteins or only the major ones (for example, the three major ones cited above). In a preferred embodiment, the egg white powder contains all the proteins present in an egg white, in approximately the same proportions as an egg white (see table below.)

| *Major proteins in the albumen of hen's egg* | | |
|---|---|---|
| protein | % of total protein | characteristics |
| Ovalbumin | 54 | Possible enzyme inhibitor and/or metal binder |
| Ovotransferrin | 12 | Iron binder |
| Ovomucoid | 11 | Protease inhibitor |
| Lysozyme | 3.4 | Enzyme: bactericide |
| Ovomucin | 1.5 | Mucilaginous virus inhibitor |
| Ovoinhibitor | 1.5 | Protease inhibitor |
| Ovoglycoprotein | 1.0 | Unknown |
| Riboflavin-binding protein | 0.8 | Protein binder |
| Ovomacroglobulin | 0.5 | Protease inhibitor |
| Thiamin-binding protein | 0.5 | Vitamin binder |
| Avidin | 0.06 | Vitamin binder |
| Cystatin | 0.05 | Protease inhibitor |
| Ovoglobulin G2 | 4 | Foaming agent |
| Ovoglobulin G3 | 4 | Foaming agent |

The calcium according to this aspect of the invention is also preferably in powder form (Ca salt). Both powders are mixed together to give a calcium-egg white powder mix or an isoflavone-egg white mix having the ability to enhance calcium absorption. The ratio egg white powder/calcium by weight can be comprised between 4 and 400, preferably 20 to 50, and in a preferred embodiment 40, as the ratio calcium/isoflavones can be comprised between 1.5 and 48, preferably 6 to 20, and in a preferred embodiment 12.

The powder mix can be used by incorporation into foods, by dissolving into water or water-containing liquids or gels, or by ways of capsules or other pharmaceutical forms.

In a variant of the first aspect of the invention, the powder mix can be supplemented with other nutrients and components, as well as with functional ingredients as defined above. For example, vitamin D can be added because it is known to facilitate calcium absorption. Other vitamins and minerals can be added as well, such as for example vitamin A, B2, B6, B12, C, E, or K and minerals such as phosphorus, potassium, sulfur, sodium, chloride, magnesium, manganese, copper or iodine and zinc among others. Preferably, vitamin D and zinc are added.

In another variant of the first a spect of the invention, prebiotics can also be added to the powder mix or the supplemented powder mix or the final product. Suitable prebiotics include oligosaccharides, such as inulin and its hydrolysis products commonly known as fructooligosaccharides, galacto-oligosaccarides, xylooligosaccharides or oligo derivatives of starch. The prebiotics may be provided in any suitable form. For example, the prebiotic may be provided in the form of plant material, which contains the prebiotic. Suitable plant materials include asparagus, artichokes, onions, wheat or chicory, or residues of these plant materials. Alternatively, the prebiotic may be provided as an inulin extract. Extracts from chicory are particularly suitable. Suitable inulin extracts are commercially available.

In the second aspect of the invention, there is provided a food product comprising egg white and calcium or isoflavones and calcium, and having the ability to enhance calcium absorption. Such food product can he a liquid food product, such as fruit juice, milk or milk-containing beverage, syrup, water, soda, oil, vinegar, sauces, and the like. It can also be a gelled product, an emulsion, tablets, a solid food product, or any other form ingestible with or without liquid. Examples of solid food products can be chocolates, chocolate powders, candies, cakes, biscuits, waffles, cereal bars, dairy products including yogurts, crèmes, and the like, breakfast cereals, pastas, purées or compotes, among other. It can be dehydrated food products, such as dehydrated soups or sauces, frozen food products, canned food products, and more generally any food product which has been industrially manufactured and/or transformed. The food product can in particular be a dietary supplement as well as a dietary additive.

The food product according to this aspect of the invention contains egg white and calcium, or isoflavones and calcium. If the food product in which the powder mix is incorporated already contains either egg white or calcium such as milk, or milk derived components, for example, the amount of the ingredient in the powder mix should be adjusted accordingly. Depending on the storage conditions and the type of product, the amount of powder mix should be comprised between 0.5 g and 45 g/serving, preferably between 1 and 30, and in a preferred embodiment 12. The ratio, in the food product, of egg white/calcium should be comprised between 4 and 400, preferably 20 to 60, and in a preferred embodiment 40, and the ratio calcium/isoflavones can be comprised between 1.5 and 48, preferably 6 to 20, and in a preferred embodiment 12.

However, it has to be understood that in another embodiment, the food product can fulfil the invention even though there is no mix powder in it, if it fulfils the ratio and amount conditions. For example, the food product can be a two chocolates sandwich cake, the dark chocolate part containing egg white or egg white proteins and the milk chocolate part containing calcium. Another example can be given with pick and croq products type: the finger biscuits can contain egg white or egg white proteins, and the cheese part the calcium. Such food products have the ability to enhance calcium absorption.

A clinical study was done to compare the effect of egg white and two milk proteins, Ca-CGMP and K-CGMT, on Ca absorption in humans. In the randomised crossover trial 20 healthy young men consumed each of the proteins (10 g) once in a drink together with a light breakfast. Fractional Ca absorption from the test meal was determined using the dual stable isotope technique by measuring oral and intravenous Ca isotopes in urine pools collected from 0-24 h following isotope administration.

Fractional absorption from egg white protein was significantly higher (31.0±1.2 %) than from Ca-CGMP (26.8±1.2 %) and K-CGMP (27.1±1.4 %).

In the third aspect of the invention, there is provided a food product comprising egg white and calcium or isoflavones and calcium, and having the ability to decrease obesity. Such food product can be a liquid food product, such as fruit juice, milk or milk-containing beverage, syrup, water, soda, oil, vinegar, sauces, and the like. It can also be a gelled product, an emulsion, tablets, a solid food product, or any other form ingestible with or without liquid. Examples of solid food products can becereal bars, hyperproteinated products such as cakes, biscuits or waffles, dairy products including yoghurts, crèmes, and the like, breakfast cereals, pastas, purées or compotes, among others. It can be dehydrated food products, such as dehydrated soups or sauces, frozen food products, canned food products, and more generally any food product which has been industrially manufactured and/or transformed. The food product can in particular be a dietary supplement as well as a dietary additive.

The food product according to this aspect of the invention contains egg white and calcium or isoflavones and calcium. If the food product in which the powder mix is incorporated already contains either egg white or milk, or milk derived components, the amount of the ingredient in the powder mix should be adjusted accordingly. Depending on the storage conditions and the type of product, the amount of powder mix should be comprised between 1 g and 30 g/serving The ratio, in the food product, of egg white/calcium should be comprised between 4 and 400, preferably 40, and the ratio calcium/isoflavones can be comprised between 1.5 and 48, preferably 6 to 20, and in a preferred embodiment 12.
In this aspect of the invention, as in the second one, the foodstuff does not have to comprise the mix powder, but can comprise egg white or egg white proteins in one part and calcium in a separate part, if the whole foodstuff is in the aforementioned ratios.

Zemel et al in their article "Regulation of adiposity by dietary calcium" (The FASEB Journal, June 2000, Vol 14, pages 1133 to 1138) disclose that increasing adipocyte intracellular Ca²⁺ result in a coordinated stimulation of lipogenesis and inhibition of lipolysis. Thus, increasing, dietary calcium suppresses adipocyte intracellular Ca²⁺ and thereby modulates energy metabolism and attenuates obesity risk. Indeed, their data indicate that, for any given level of energy intake and expenditure, a low calcium diet favours increased adipose tissue energy storage, but the converse is true for higher calcium diets. Accordingly, dietary calcium appears to modulate the efficiency of energy utilization, with low calcium diets favouring increased efficiency of energy storage and higher calcium diets reducing energy efficiency and instead favouring increased thermogenesis.
Davies et al, in an article titled. Calcium intake and body weight (The Journal of clinical endocrinology & metabolism, Vol 85, n°12, pages 4635 to 4638) confirmed the results of Zemel.

Consequently, increasing calcium absorption, which is an aspect of our invention, allows attenuation of obesity. According to this aspect of the invention, we give together calcium and egg white or egg white proteins, or calcium and isoflavones in order to decrease obesity, as well as to increase weight loss, help reducing weight and/or to reduce body fat.

By losing weight, human beings and pets cause less damage to their joints, especially to the ankle, knee and hip joints, which have to support the body weight. In parallel, it is easier for the muscles to move the body mass as this mass has been decreased. And myocardium, which is one of the most important muscles, is less strained by everyday efforts. All these effects play a role on mobility: usually, obese humans and pets are not very mobile and when they move they are tired very rapidly. By enhancing calcium absorption and decreasing obesity, or by enhancing calcium absorption and reducing the body weight, mobility of the pet or of the human is increased.

According to another aspect of the invention, there is provided a composition of calcium and egg white or egg white proteins to increase bone mass.

It has to be understood that the combination of isoflavones/calcium and that food products containing such a combination are preferably intended for women facing menopause or having a high probability to face it in the following years.

### Examples

The following examples are illustrative of' some of the products and methods of making the same falling within the scope of the present invention. They are not to be considered in any way limitative of the invention. Changes and modifications can be made with respect to the invention. That is, the skilled person will recognise many variations in these examples to cover a wide range of formulas, ingredients, processing, and mixture to rationally adjust the naturally occurring levels of the compounds of the invention for a variety of applications.

### Example 1: cereals to enhance calcium absorption

A dry mixture and a binder are prepared using the following ingredients (calculated in percent by weight):

| Binder | |
|---|---|
| Glucose syrup | 4 |
| Sucrose | 4 |
| Invert sugar | 10 |
| White egg (powder) | 19.47 |
| Fat | 7.82 |
| Lecithin | 0.1 |

| Dry mixture | |
|---|---|
| Apple cubes | 7 |
| Calcium | 0.53 |
| Rice crisp | 10 |
| Oat bran | 30 |
| Oat bran concentrate | 6.0 |
| Guar | 1 |
| Apple flavour | 0.08 |

The percentages of guar, oat bran and oat bran concentrate, in weight percent of the dry mix, are 1.9%, 18.9% and 7.5%, respectively.

The rice crisps were obtained by cooking-extruding-expanding rice flour according to standard techniques, purchased from GEMEF Industries, 44, rue du Louvre, Paris, France.

The ingredients are thoroughly mixed and put into a Bepex-Hutt Roller Slab Former Type GP, which presses the mixture and yields an about 1.5cm thick, flat slab. Then pieces of about 20g are cut out from the pressed paste to obtain longish bars.

### Example 2: soup to decrease obesity

The following ingredients in powder form are mixed together in a HOBART mixer.

| | |
|---|---|
| Fine salt | 50 g |
| Glutamate | 20 g |
| Sugar | 60 g |
| Calcium | 0.23 g |
| Tomato powder | 200 g |
| Potato starch | 100 g |
| Wheat flour | 200 g |
| Egg white powder | 8.10 g |
| Onion powder | 10 g |
| Garlic powder | 5 g |

The ingredients in powder form of the above-mentioned mixture have a mean size of 120 micro metres. The fats (330g of hydrogenated palm oil) are melted at a temperature of approximately 40°C. The powder mix is added to the fats and the whole mixture is mixed and homogenized at a temperature of 40°C in a thermostated mixer.
This mixture is flowed in rectangular moulds of 9 cm long, 5 cm wide and 0.7 cm thick. The moulds, once they are filled, are cooled in a refrigerated closet at a temperature of 4°C. Tablets are then demoulded by application of vibrations.

This gives a tomato soup in table form giving calcium and allowing and suitable for obese people, as it helps lowering (reducing?) obesity.

### Example 3: cat food to enhance absorption

We prepare an emulsion with 63% meat and meat by-products, in particular with poultry by-products (mainly carcass) and/or pork or beef by-products (mainly liver and lungs), 15% cereals, 1.5% texturing proteins from animal or vegetal origin, 0.2% egg white proteins, 5.2% calcium and 1.5% water. The emulsion also contains vitamins, salt, aromas and colourings.
The emulsion is then cooked in an oven. The chews obtained are very efficient to allow a higher absorption of calcium in cats.

### Example 4: infant formula to enhance calcium absorption

We prepare an infant formula by mixing together the following ingredients in the indicated proportions. The final product is in powder form.

| | |
|---|---|
| Fat | 27.7g |
| Fat from milk | 0.7g |
| Mix of fats (150) | 26.8 g |
| Lecithin | 0.2g |
| Linoleic acid | 4.1 g |
| Alpha-linolenic acid | 52.5 mg |
| Proteins | 9.5 mg |
| Available carbohydrates | 57.9 g |
| Lactose | 57.9 g |
| Minerals (ashes) | 1.9 g |
| Sodium | 120 mg |
| Potassium | 460 mg |
| Chlorure | 330 mg |
| Calcium | 320 mg |
| Phosphorus | 160mg |
| Magnesium | 36 mg |
| Manganese | 40 micro grams |
| Selenium | 10.4 micro grams |
| Total solids | 97.0 g |
| Humidity | 3.0 g |

13.12 grams of proteins from egg white are added to the above-mentionned formula.

Such an infant formula enhances absorption of calcium in the infant or child who drinks it.

### Example 5: results of the experiment on isoflavones and calcium absorption.

Fifty-five 9-10 month old female Wistar rats (model of menopausal bone loss), were used to test whether soy isoflavones enhance calcium balance. The animals were first equilibrated to a small rodents special diet containing 0.3% Ca and 0.2% P for 3 weeks. One week prior to ovariectomy, they were randomised based on body weight and 6 groups were formed (*n*=8 per group). 40 rats were then ovariectomised and 8 were SHAM operated (i.e. surgery without ovariectomy). The 2 control groups (SHAM and OVX) continued to consume the equilibrium diet and the remaining ovariectomised rats received the equilibrium diet supplemented with isoflavones at physiological doses. The isoflavones were given either in the glycoside (Novasoy, ADM) or aglycone (enzymatically hydrolyses Novasoy) form and at two different doses (3 mg or 8 mg per day). Blood samples were taken on days 1, 47, 85 and calcium balance was assessed on days 43 to 47 and days 78 to 82.

As expected, calcium balance deteriorated in all 6 groups over the 2-month period which was probably due to the strong effects of estrogen depletion and aging on calcium absorption and retention.

Over a 2-month period, which is equivalent to about 4 years in human terms, daily consumption of soy isoflavones tended to enhance Ca balance in all isoflavone groups compared to the OVX controls. The isoflavone aglycones and glycosides gave equivalent plasma isoflavone levels (due to efficient conversion of glycosides to aglycones in the rat gastrointestinal tract) and the differences in doses (3 mg or 8 mg/day) did not strongly influence the final steady-state plasma concentration (around 5 µM). Curiously, the low dose aglycone group showed the most significant improvement in Ca balance.
In this animal model of menopausal bone loss, daily consumption of soy isoflavones for 2 months enhanced Ca balance compared to controls

### Example 6: High calcium milk for post menopausal women containing isoflavones to increase calcium absorption.

A high calcium milk for post menopausal women is prepared by mixing together the following ingredients:
111 grams of partially defatted milk powder,
1 L of water,
800 mg of calcium carbonate,
100 mg of isoflavones.

Recommended daily intake of this milk can vary from 250 mL to 500 mL a day.

### Example 7: juice containing isoflavones and egg white proteins

Cartons of orange juice with a capacity of 250 mL are prepared by mixing the following ingredients:
200 mg of calcium carbonate,
4 grams of egg white proteins, and
33 mg of isoflavones.

Orange juice is added under stirring conditions until the final desired volume is reached.

### Embodiments

1. Calcium absorption enhancer comprising as an active ingredient calcium and at least one of the components selected from the group of egg white, egg white proteins and isoflavones.
2. Calcium absorption enhancer according to claim 1 wherein the egg white proteins are in their natural form.
3. Calcium absorption enhancer according to claim 1 or 2 wherein the egg white or the egg white proteins contains only ovalbumin, ovotransferrin, and ovomucoid.
4. Calcium absorption enhancer according to one of claims 1 to 3 wherein the ratio of egg white/calcium is comprised between 4 and 400, preferably 20 to 60, and in a preferred embodiment 40.
5. Calcium absorption enhancer according to one of claims 1 to 4 wherein the ratio calcium/isoflavones is comprised between 1.5 and 48, preferably 6 to 20, and in a preferred embodiment is 12.
6. Orally ingestible; composition comprising a calcium absorption enhancer according to any of the preceding claims.
7. Orally ingestible composition according to claim 6 wherein the composition is a food product, a dietary supplement, a nutritional supplement or a pharmaceutical.
8. Orally ingestible composition according to claim 6 or claim 7 wherein the proportion of egg white and calcium compared to the total composition is from 0.2 to 100% in weight, preferably 1 to 99% and in a preferred embodiment 5 to 98%.
9. Orally ingestible composition according to one of claims 6 to 8 further comprising prebiotics and/or functional ingredients.
10. Orally ingestible composition according to one of claims 6 to 9 wherein the food product is a human food product or a pet food product.
11. Orally ingestible composition according to one of claims 6 to 10 wherein the active ingredients are in powder form.
12. Use of a calcium absorption enhancer according to any of claims 1 to 5.
13. Use of a calcium absorption enhancer according to claim 12 wherein the ratio of egg white/calcium is comprised between 4 and 400, preferably 20 to 60, and in a preferred embodiment 40.
14. Use of a calcium absorption enhancer according to claim 12 or 13 wherein the ratio calcium/isoflavones is comprised between 1.5 and 48, preferably 6 to 20, and in a preferred embodiment is 12.
15. Use of a calcium absorption enhancer according to one of claims 12 to 14 wherein calcium and egg white are given at the same time but separately.
16. Use of a calcium absorption enhancer according to one of claims 12 to 15 to attenuate obesity.
17. Use of a calcium absorption enhancer according to claim 16 for pets or for humans.
18. Use of a calcium absorption enhancer according to one of claims 12 to 17 to higher bone mass.
19. Use of a calcium absorption enhancer according to one of claims 12 to 18 to higher the bone mass peak.
20. Use of a calcium absorption enhancer according to claims 12 to 19 to delay the arrival in the critical, bone mass.
21. Use of a calcium absorption enhancer according to claim 12 to 20 to increase weight loss, help reducing weight and/or to reduce body fat.
22. Use of a calcium absorption enhancer according to claims 12 to 21 to increase mobility.

## Claims

1. Calcium absorption enhancer comprising as an active ingredient calcium and at least one of the components selected from the group of egg white, egg white proteins and isoflavones, for the preparation of a composition to decrease density

2. according to claim 1 wherein the egg white proteins are in their natural form.

3. according to claim 1 or 2 wherein the egg white or the egg white proteins contains only ovalbumin, ovotransferrin, and ovomucoid.

4. according to one of claims 1 to 3 wherein the ratio of egg white/calcium is comprised between 4 and 400, preferably 20 to 60, and in a preferred embodiment 40.

5. according to one of claims 1 to 4 wherein the ratio calcium/isoflavones is comprised between 1.5 and 48, preferably 6 to 20, and in a preferred embodiment is 12.

6. Use of a calcium absorption enhancer according to claim 1-5 for pets or for humans.

7. Use of a calcium absorption enhancer according to claim 1-6 to to increase weight loss, help reducing weight and/or to reduce body fat.

8. Use of a calcium absorption enhancer according to claim 1-7 to increase mobility.
